(19) Europäisches Patentamt
European Patent Office
Office européen des brevets

(11) **EP 1 871 227 B1**

(12) **EUROPÄISCHE PATENTSCHRIFT**

(45) Veröffentlichungstag und Bekanntmachung des Hinweises auf die Patenterteilung:
**27.05.2009 Patentblatt 2009/22**

(21) Anmeldenummer: **06707536.6**

(22) Anmeldetag: **13.03.2006**

(51) Int Cl.:
**A61B 5/103** *(2006.01)* **A61B 5/107** *(2006.01)*

(86) Internationale Anmeldenummer:
**PCT/EP2006/002287**

(87) Internationale Veröffentlichungsnummer:
**WO 2006/094833 (14.09.2006 Gazette 2006/37)**

(54) **VERFAHREN ZUM BESTIMMEN DER INKLINATION UND ANTEVERSI0N EINER HÜFTPFANNE ANHAND VON ZWEI RÖNTGENAUFNAHMEN**

METHOD FOR DETERMINING A COTYLOID CAVITY INCLINATION AND ANTEVERSION USING TWO X-RAY PICTURES

PROCEDE POUR DETERMINER L'INCLINAISON ET L'ANTEVERSION D'UNE CAVITE COTYLOIDE EN FONCTION DE DEUX PRISES DE VUE RADIOGRAPHIQUES

(84) Benannte Vertragsstaaten:
**DE ES FR GB IT**

(30) Priorität: **11.03.2005 DE 102005012708**

(43) Veröffentlichungstag der Anmeldung:
**02.01.2008 Patentblatt 2008/01**

(73) Patentinhaber: **Eberhard-Karls-Universität Tübingen**
**72076 Tübingen (DE)**

(72) Erfinder: **MÜLLER, Otto**
**72393 Burladingen (DE)**

(74) Vertreter: **Wegener, Markus et al**
**Witte, Weller & Partner**
**Patentanwälte**
**Postfach 10 54 62**
**70047 Stuttgart (DE)**

(56) Entgegenhaltungen:
- **MÜLLER O ET AL: "[Quantification and visualization of the influence of pelvic tilt upon measurement of acetabular inclination and anteversion]" ZEITSCHRIFT FÜR ORTHOPÄDIE UND IHRE GRENZGEBIETE. 2005 JAN-FEB, Bd. 143, Nr. 1, Januar 2005 (2005-01), Seiten 72-78, XP009068819 ISSN: 0044-3220**
- **MURRAY D W: "The definition and measurement of acetabular orientation." THE JOURNAL OF BONE AND JOINT SURGERY. BRITISH VOLUME. MAR 1993, Bd. 75, Nr. 2, März 1993 (1993-03), Seiten 228-232, XP002388822 ISSN: 0301-620X in der Anmeldung erwähnt**
- **VISSER J D ET AL: "A new method for measuring angles after total hip arthroplasty. A study of the acetabular cup and femoral component." THE JOURNAL OF BONE AND JOINT SURGERY. BRITISH VOLUME. 1981, Bd. 63B, Nr. 4, 1981, Seiten 556-559, XP002388824 ISSN: 0301-620X in der Anmeldung erwähnt**
- **LEMBECK BURKHARD ET AL: "Pelvic tilt makes acetabular cup navigation inaccurate." ACTA ORTHOPAEDICA. AUG 2005, Bd. 76, Nr. 4, August 2005 (2005-08), Seiten 517-523, XP002388806 ISSN: 1745-3674**

**Beschreibung**

Bei Hüftendoprothesen ist eine korrekte Orientierung der Hüftpfanne für den langfristigen Erfolg von Hüftendoprothesen eine wichtige biomechanische Voraussetzung. Die Orientierung der Hüftpfanne im Raum wird (klinisch) durch zwei Winkelgrößen angegeben, und zwar durch die Inklination und die Anteversion.

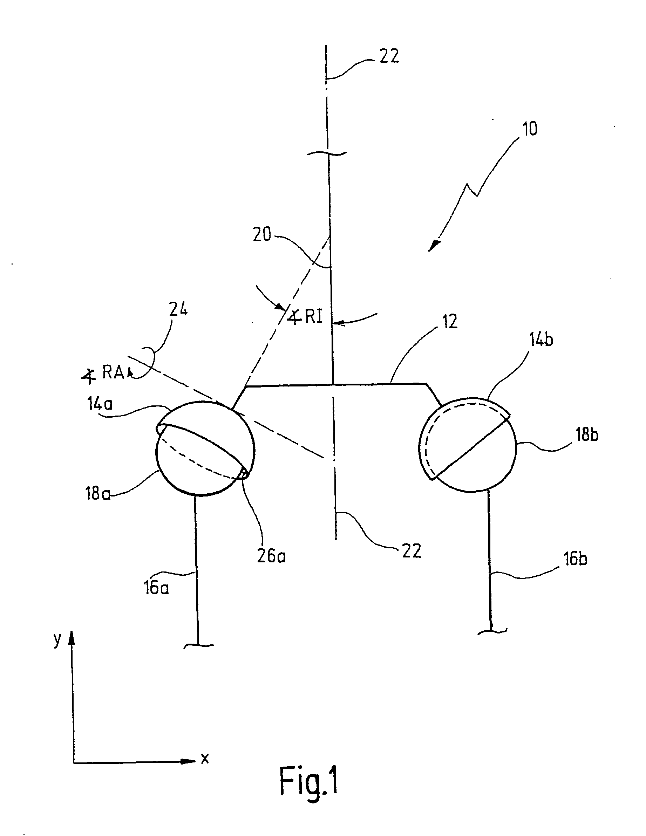

In Fig. 1 ist ein Teil eines menschlichen Skeletts stark schematisiert dargestellt, wobei im Wesentlichen lediglich der Bereich eines Beckens 10 in einer sog. Frontalebene bzw. eine aprior-posterior-Beckenübersichtsansicht (a.p.-BÜS) dargestellt ist.

Fig. 1 zeigt schematisch einen Beckenknochen 12, der eine linke Hüftpfanne 14a und eine rechte Hüftpfanne 14b umfasst. In den als Halbkugeln idealisiert dargestellten Hüftpfannen 14a und 14b sind Hüftknochen 16a und 16b mit entsprechenden (idealisiert kugelförmigen) Hüftköpfen 18a und 18b gelagert. Die Wirbelsäule ist mit dem Bezugszeichen 20 bezeichnet. Die Fortsetzung der Wirbelsäule 20 stellt die Körperlängsachse 22 dar, die in Fig. 1 mit einer strichpunktierten Linie dargestellt ist.

Entlang der Körperlängsachse 22 und senkrecht zur Zeichenebene der Fig. 1 spannt sich die sog. Sagittalebene. Die Zeichenebene entspricht hier der Frontalebene. Senkrecht zur Sagittalebene und zur Frontalebene spannt sich die sog. Transversalebene. Die Frontalebene entspricht i.d.R. einer Draufsicht auf einen Patienten von vorne. Die (tatsächliche) linke bzw. rechte Hüfte des Patienten ist deshalb in der Frontalebene rechts bzw. links zu sehen.

Die Inklination stellt den Wert der Abduktion der Hüftpfanne 18 dar. Unter der Abduktion versteht man den Winkel zwischen der Hüftpfanne 14 und der Körperachse 22 in der Frontalebene.

Die Orientierung der Hüftpfanne 14 wird des Weiteren durch einen Winkel für die sog. Anteversion angegeben, der einen Wert für die Rotation der Hüftpfanne um eine Achse in der Frontalebene repräsentiert. Diese Rotation ist in Fig. 1 durch einen Pfeil 24 angedeutet. Um diese Rotation besser veranschaulichen zu können, ist die in der Fig. 1 links angeordnete Hüftpfanne 14a perspektivisch dargestellt, so dass man, im Gegensatz zu der zweidimensional dargestellten Hüftpfanne 14b, eine Öffnungsfläche 26a der Hüftpfanne 14a erkennen kann.

Werte von 45 ± 10 Grad für die Inklination und 15 ± 10 Grad für die Anteversion haben sich als vorteilhaft erwiesen. Diese Winkelbereiche werden, bezogen auf das körpereigene (anatomische) Bezugssystem, bei Hüftoperationen für die Hüftendoprothese angestrebt. Beide Größen stellen Winkelangaben dar, welche die Ausrichtung der Pfannenebene 26 in Relation zu den anatomischen Referenzebenen (Frontalebene, Sagittalebene, Transversalebene) mathematisch charakterisieren. Diese Winkel können jedoch nicht nur auf ein anatomisches Bezugssystem bezogen angegeben werden, sondern auch auf ein äußeres Bezugssystem, wie z.B. ein Röntgengerät oder ein Operationsbezugssystem. Verschiedene Definitionen für die Inklination und die Anteversion sind in dem Artikel "The Definition and Measurement of Acetabular Orientation" von D. W. Murray in "THE JOURNAL OF BONE AND JOINT SURGERY", Seite 228 bis 232, Vol. 75-B, Nr. 2, März 1993 angegeben. Die dort angegebene Nomenklatur sowie die verschiedenen Definitionen werden nachfolgend auch hier verwendet werden.

Die Inklination als auch die Anteversion stellen also Winkelgrößen dar, welche die Ausrichtung der Pfannenebene in Relation zu den anatomischen Referenzebenen (Sagittalebene, Frontalebene, usw., s. Murray) mathematisch charakterisieren. Im klinischem Alltag wird versucht, beide Größen aus konventionellen Röntgenaufnahmen zu ermitteln.

Sowohl präoperativ zur Operationsplanung als auch postoperativ zur Beurteilung des Operationserfolgs sind diese beiden Winkelgrößen für den Orthopäden wichtige Messgrößen, da die Werte für die Inklination vorzugsweise in einem Bereich von 45 Grad ± 10 Grad und für die Anteversion in einem Bereich von 15 Grad ± 10 Grad liegen sollen.

Die Bestimmung dieser Winkelgrößen an Hand konventioneller Röntgentechniken bringt zwei gravierende Nachteile mit sich, die eine exakte Bestimmung der Inklination und der Anteversion erschweren bzw. unmöglich machen.

Ein erster Nachteil ist darin zu sehen, dass sich die zu bestimmenden Winkel auf geometrische Linien im Raum, insbesondere auf geometrische Strukturen am Becken, beziehen. Im klinischen Alltag wird versucht, diese Winkel aus konventionellen Röntgenbildern zu ermitteln. An Hand dieser Bilder sind die Winkel aber nur unzureichend bestimmbar, da die Winkel im Raum auf ebenen Projektionsflächen in der Regel "verzerrt" abgebildet werden. Ein Grund für diese Verzerrung ist darin zu sehen, dass die Projektionsebene, d.h. die Ebene eines Röntgenbilds, üblicherweise nicht der anatomischen Ebene entspricht, in der die Winkel definiert sind.

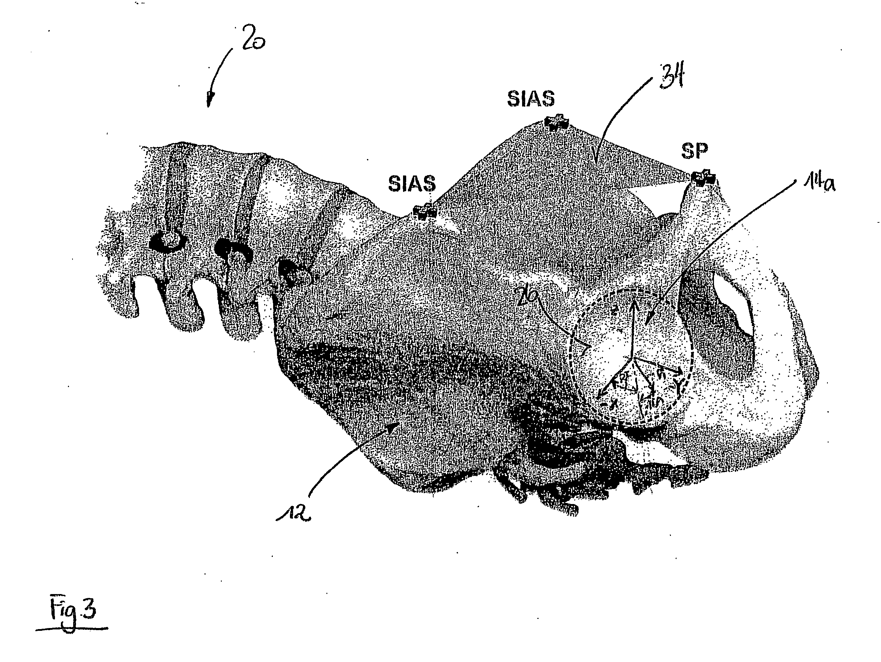

Ein zweiter Nachteil ist darin zu sehen, dass die Angabe der Inklination und der Anteversion mathematisch, und auch technisch, nur dann Sinn macht, wenn sie sich auf ein beckeninternes Referenzsystem, d.h. ein anatomisches Koordinatensystem, beziehen. Das anatomische Beckenkoordinatensystem wird an Hand von drei markanten Punkten am Beckenknochen definiert, die eine sog. Beckeneingangsebene bilden, wie es im Zusammenhang mit Fig. 3 noch detaillierter beschrieben werden wird.

Herkömmliche Auswerteverfahren, wie jenes von Sven-Johansson, oder die Visser-Technik (Johansson, S., "Zur Technik der Osteosynthese der Fratt. coli femoris, Zbl. Chir., 59, 2019 - 2022, 1932; Visser, J.D. et al., 1981, A New Method for Measuring Angles After Total Hip Arthroplasty, J. Bone Joint Surg. Br., 63B, 556 - 559), orientieren sich nicht am anatomischen Referenzsystem, sondern an einem Koordinatensystem, welches durch die Richtung des Röntgenstrahls und die Orientierung der Projektionsebene des Röntgenbildes dazu definiert ist. Die Standardaufnahme zur Beurteilung eines Beckens ist die sog. anterior-posteriore Beckenübersichtsaufnahme. Der Röntgenstrahl fällt dabei frontal auf den Patienten ein. Das Röntgenprojektionsbild ist dann parallel zur Frontalebene des Patienten orientiert.

Durch eine definierte Patientenlagerung wird versucht, den ersten beschriebenen Nachteil zu beseitigen bzw. dessen Fehlerquellen so klein wie möglich zu halten. Der zweite beschriebene Nachteil ist jedoch systemimmanent, da die Orientierung der Beckeneingangsebene bei den oben genannten Methoden nicht berücksichtigt wird.

Bei der Sven-Johansson-Technik sind zwei Röntgenaufnahmen erforderlich, zwischen denen der Patient jedoch umgelagert werden muss. Dies bedeutet, dass die Orientierung des Patientenbeckens im Raum zwischen den Aufnahmen geändert wird. Die Sven-Johansson-Technik erfordert eine a.p.-Beckenübersichtsaufnahme (BÜS) sowie eine axiolaterale Röntgenaufnahme, wobei der Röntgenstrahl in der Frontalebene um 45° zur Längsachse gedreht verläuft und das Gegenbein angehoben wird. Dies stellt an die Patientenlagerung sehr spezielle Anforderungen, die im klinischen Alltag selten eingehalten werden können.

Die Visser-Technik analysiert die Hauptachsen eines projizierten Pfannenrands an Hand eines einzigen Röntgenbildes (nämlich der a.p.-BÜS) und setzt dabei implizit voraus, dass die Beckeneingangsebene parallel zur Projektionsebene des Röntgenbildes, d.h. der Frontalebene bzw. dem Röntgenfilm, orientiert ist. Dabei wird die Anteversion einer implantierten Metallpfanne aus dem Hauptachsenverhältnis des als Ellipse projizierten Pfannenrandes bestimmt.

Beide Verfahren berücksichtigen eine Verkippung der Beckeneingangsebene gegenüber der Frontalebene nicht. Es lässt sich jedoch rechentechnisch nachweisen, dass die Verkippungen der Beckeneingangsebene gegenüber der Frontalebene, die zwangsläufig beim Erstellen der Röntgenbilder entstehen, zu Fehlern von bis zu $\pm$ 10 Grad bei der Bestimmung der Inklination und/oder der Anteversion führen können.

Zwar wäre die Bestimmung der Inklination und der Anteversion unter Verwendung von Schichtaufnahmen mittels Computertomographie ("CT") prinzipiell möglich. Jedoch wird diese Möglichkeit in der Praxis nicht umgesetzt, da CT-Aufnahmen mit erheblichen Strahlenbelastungen für den Patienten und ferner mit höheren Kosten verbunden sind.

Auch ist es möglich, während der Operation rechnergestützte Navigationssysteme zu verwenden, die entwickelt wurden, um den Chirurgen während der Implantierung der Hüftpfanne zu unterstützen und zu führen. Unabhängig davon, ob diese Navigationssysteme mit CT-Bildern oder einer CT-freien Technik arbeiten, müssen anatomische Markierungspunkte des Beckens intraoperativ festgestellt werden, um das anatomische Koordinatensystem zu definieren. In einem solchen Fall wird die Orientierung der Hüftpfanne mit einem sog. Pfannenfräser angepasst, der durch ein Führungssystem in Bezug auf das so erhaltene anatomische Koordinatensystem geführt wird.

Ungeachtet der mit diesen Navigationssystemen erreichbaren Genauigkeit hängt die exakte Ausrichtung der Hüftpfanne dennoch stark davon ab, wie exakt die Markierungen in Bezug auf den Beckenknochen gesetzt werden. Deshalb ist es weiterhin wünschenswert, die Ausrichtung der Hüftpfanne auch postoperativ zu bestimmen. Jedoch berücksichtigt keines der herkömmlichen Röntgenverfahren das anatomische Koordinatensystem des Beckens. Messfehler in der Größenordnung von 10 Grad wurden bei Computersimulationen für die Anteversion festgestellt, was jedoch von der Ausprägung der Beckenneigung abhängt, die sich in einem Bereich von ungefähr 20 Grad hinsichtlich der Reklination und 10 Grad hinsichtlich der Inklination bewegen kann.

Es ist deshalb eine Aufgabe der vorliegenden Erfindung, ein verbessertes Verfahren zum genauen Bestimmen der Inklination und Anteversion, insbesondere in Relation zu anatomischen Referenzebenen, bereitzustellen, wobei eine Winkelgenauigkeit von besser als $\pm$ 5 Grad gewünscht ist.

Diese Aufgabe wird durch ein Verfahren gelöst, das die folgenden Schritte aufweist: Bereitstellen eines ersten Bilds des Beckenknochens, wobei das erste Bild eine Frontalebene mit dem Beckenknochen zeigt; Bestimmen eines ersten Inklinationswinkels der Hüftpfanne unter Verwendung des ersten Bilds; Bestimmen eines Beckenrotationswinkels, der den Rotationswinkel des Beckenknochens um die Normale der Frontalebene relativ zu einer optimalen Patientenlagerungsposition angibt; Bereitstellen eines zweiten Bilds des Beckenknochens, wobei die Ebene des zweiten Bilds um einen Bildrotationswinkel relativ zu der Frontalebene um eine gemeinsame Schnittlinie der beiden Ebenen herum, vorzugsweise um die Körperlängsachse des Patienten herum, rotiert ist; Bestimmen eines zweiten Inklinationswinkels der Hüftpfanne unter Verwendung des zweiten Bilds; Bestimmen eines Beckenverkippungswinkels, der eine Rotation der Frontalebene des ersten Bildes relativ zu einer Beckeneingangsebene in der optimalen Patientenlagerungsposition repräsentiert; Aufstellen eines linearen Gleichungssystems mit mindestens zwei linearen Gleichungen in Abhängigkeit von den zuvor bestimmten Winkeln; Ausdrücken des Normalenvektors $n_0$ in Polarkoordinaten des anatomischen Bezugssystems; Auflösen der zumindest zwei linearen Gleichungen nach Azimuth und Polarwinkel des Normalenvektors $n_0$, um so die Inklination und die Anteversion zu bestimmen.

Das erfindungsgemäße Verfahren hat den Vorteil, dass lediglich zwei Bilder des Beckenknochens des Patienten erzeugt werden müssen, was eine Zeitersparnis gegenüber dem CT darstellt. Ferner werden die Patienten einer geringeren Strahlenbelastung ausgesetzt, wenn die Bilder basierend auf Röntgenstrahlen erzeugt werden.

Außerdem berücksichtigt das Verfahren gemäß der vorliegenden Erfindung die Beckenverkippung, wodurch genauere Aussagen über die Orientierung der Hüftpfanne bzw. die Inklination und Anteversion im anatomischen Bezugssystem getroffen werden können.

Diese genaueren Aussagen sind sowohl präoperativ als auch postoperativ von großer Bedeutung. Bei der präoperativen Planung einer Hüftoperation lassen sich genauere Aussagen über die Orientierung der ursprünglichen Hüftpfannen treffen. Bei der postoperativen Kontrolle lassen sich somit genauere Aussagen über den Erfolg bzw. den Misserfolg einer Operation treffen.

Gemäß einer bevorzugten Ausführungsform der Erfindung entspricht das Gleichungssystem einer linearen Abbildung A, die wiederum den Normalenvektor der Hüftpfannenöffnungsebene auf den Nullvektor des anatomischen Bezugssystems abbildet.

Ferner ist es von Vorteil, wenn alternativ zum Auflösen der zumindest zwei linearen Gleichungen nach Azimuth und Polarwinkel des Normalenvektors, der Azimuth und der Polarwinkel des Normalenvektors durch Bestimmen des Eigenvektors der Abbildung A beim Eigenwert Null bestimmt wird.

Besonders von Vorteil ist es, wenn eine erste Gleichung der linearen Gleichung, eine Drehung der Hüftpfanne um den Beckenverkippungswinkel α und um den Beckenrotationswinkel δ zum Darstellen des ersten Inklinationswinkels RI2 repräsentiert und eine zweite Gleichung der linearen Gleichung eine weitere Drehung der ersten Gleichung um den Bilddrehwinkel β zum Darstellen des zweiten Inklinationswinkels RI3 repräsentiert.

Eine optimale Patientenlagerungsposition ist dadurch definiert, dass die Beckeneingangsebene parallel zur Frontalebene orientiert ist und dass eine Verbindungslinie der Hüftpfannenmittelpunkte parallel zu einem der Einheitsvektoren orientiert ist, der die Frontalebene aufspannt, wobei der andere Einheitsvektor parallel zur Körperlängsachse des Patienten orientiert ist.

Diese Maßnahme erleichtert das Bestimmen der Winkel aus den Bildern, wobei das bildgebende System um die Körperlängsachse rotiert wird. Der Beckenrotationswinkel δ kann somit mitunter Null sein.

Gemäß einer weiteren bevorzugten Ausführungsform ist das anatomische Bezugssystem durch die Beckeneingangsebene definiert, welche durch die Spinae am vorderen Beckenkamm sowie die Symphysenfuge des Beckenknochens definiert ist.

Ferner ist es von Vorteil, wenn das bildgebende System zur Erzeugung des zweiten Bildes um die Körperlängsachse des Patienten rotiert wird.

Bevorzugterweise werden der erste und der zweite Inklinationswinkel jeweils mittels einer Hauptachse des Pfannenrandes bzw. eines entsprechenden Normalenvektors bestimmt, der jeweils senkrecht auf einer Öffnungsfläche der interessierenden Hüftpfanne steht, wie sie im ersten bzw. zweiten Bild abgebildet ist.

Dabei kann der jeweilige Normalenvektor bzw. dessen Projektion bestimmt werden, indem die Hüftpfanne durch eine offene Halbkugel genähert wird, wobei ein Öffnungsrand der jeweiligen Halbkugel durch eine Ellipse genähert wird, deren Hauptachsen ermittelt werden, zu denen der jeweilige (imaginäre) Normalenvektor orthogonal orientiert ist.

Der erste und zweite Inklinationswinkel kann geometrisch an Hand des jeweiligen Winkels zwischen dem Normalenvektor und der Horizontalen der Frontalebene bestimmt werden.

Außerdem kann der Ursprung der Frontalebene in den Mittelpunkt der Hüftpfanne gelegt werden.

Gemäß einer weiteren bevorzugten Ausführungsform werden die Bilder in Form von Datensätzen bereitgestellt.

Dies hat den Vorteil, dass die Bilder nach Erstellung beispielsweise digitalisiert abgespeichert werden, um später einer entsprechenden Planungs- oder Überprüfungssoftware zugeführt zu werden.

Gemäß einer weiteren Ausführungsform werden die Bilder mittels Röntgenaufnahmen oder NMR bereitgestellt.

Der Beckenrotationswinkel δ kann geometrisch als Winkel zwischen einer Horizontalen der Frontalebene und einer (imaginären) Verbindungslinie zwischen den Hüftpfannenmittelpunkten bestimmt werden.

Vorzugsweise wird der Beckenverkippungswinkel α unter Verwendung einer Beckenwaage bestimmt.

Auch ist es von Vorteil, wenn die Bilder unter Verwendung eines bildgebenden Systems bereitgestellt werden, welches eine Strahlungsquelle und eine Erfassungseinrichtung umfasst, die starr zueinander angeordnet sind und insbesondere um die Körperachse des Patienten drehbar sind. Das bildgebende System kann ein sog. C-Bogen sein.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert.

Fig. 1    zeigt eine schematisierte Draufsicht auf einen Beckenknochen und eine teilweise dargestellte Wirbelsäule sowie teilweise dargestellte Oberschenkelknochen.

Fig. 2    stellt einen Normalenvektor einer Hüftpfanne in einem anatomischen Bezugssystem dar.

Fig. 3    zeigt eine Beckeneingangsebene an Hand eines perspektivisch dargestellten Beckenknochens.

Fig. 4    zeigt einen Teil einen ersten Aufnahme einer Hüftpfanne, aus der der Normalenvektor bestimmt werden kann.

Fig. 5a und 5b      zeigen ein Beispiel für die Anordnung eines Patienten relativ zu einem bildgebenden System.

Fig. 6      zeigt ein Beispiel, wie zwei Bilder gewonnen werden können, die zur Durchführung des Verfahrens gemäß der Erfindung benötigt werden.

Mathematisch lässt sich die Orientierung einer Hüftpfanne durch einen Normalenvektor $\vec{n}$ der Hüftpfannenöffnungsebene in Bezug auf ein anatomisches Beckenkoordinatensystem repräsentieren (vgl. Fig. 1). Das anatomische Beckenkoordinatensystem wird vorzugsweise mit Hilfe der Beckeneingangsebene definiert, die wiederum durch zwei Spinae des Beckens ("spines iliac anterior superior, SIAS") und die Symphysenfuge des Beckenknochens definiert sind. Diese Ebene wird durch die x- und y-Einheitsvektoren eines kartesischen Koordinatensystems aufgespannt. Der z-Einheitsvektor dieses Systems ist normal zu dieser Ebene und kommt aus dieser heraus. Die Inklination und Anteversion der Hüftpfanne kann so in Polarkoordinaten in Form des Azimuts und des Polarwinkels ausgedrückt werden.

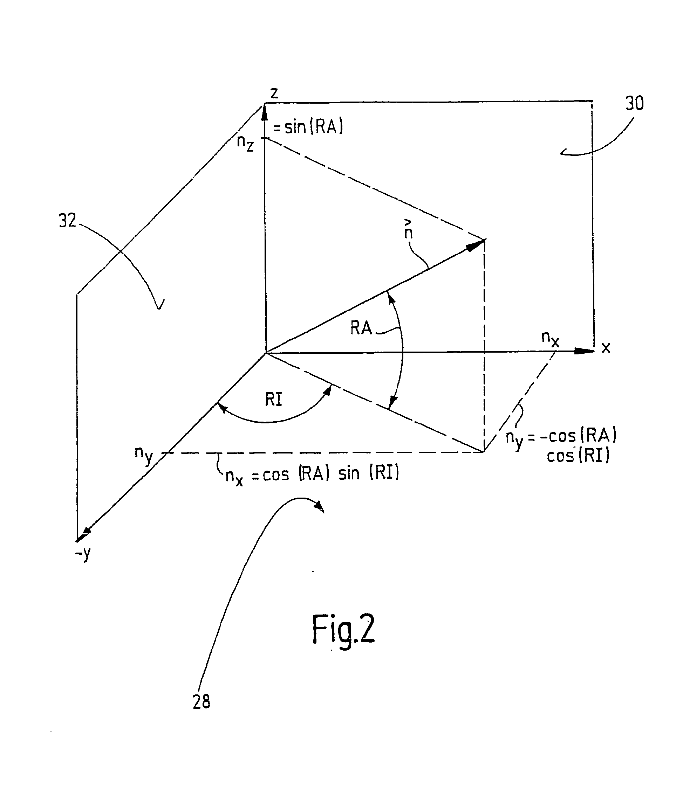

Fig. 2 zeigt exemplarisch die Orientierung eines beliebigen Vektors $\vec{n}$ in einem Polarkoordinatensystem.

Der Normalenvektor $\vec{n}$ steht senkrecht auf der Pfannenöffnungsfläche 26 (vgl. Fig. 3). Die Hüftpfanne selbst ist in Fig. 2 nicht dargestellt, um die Fig. 2 übersichtlich zu halten.

Der Ursprung des kartesischen Koordinatensystems der Fig. 2 stimmt mit dem Mittelpunkt der Hüftpfannenöffnungsfläche überein. Im Beispiel der Fig. 2 ist der Normalenvektor $\vec{n}$ der rechten Hüftpfanne 14b der Fig. 1 schematisch dargestellt. Zur Abbildung der Fig. 1 kommt man, wenn man die Fig. 2 von oben, d.h. aus der z-Richtung, betrachtet, wobei in der Fig. 2 lediglich die rechte Hüfte bzw. deren Normalenvektor dargestellt ist.

Die Einheitsvektoren des kartesischen Koordinatensystems der Fig. 2 spannen Ebenen auf, die wie nachfolgend bezeichnet werden. Die Einheitsvektoren x und y spannen die Frontalebene 28 auf. Die Frontalebene 28 ist bei klinischen Röntgenaufnahmen parallel zu der Ebene des Röntgenfilms bei der a.p.-BÜS-Aufnahme. Bei der a.p.-BÜS-Aufnahme ist der Patient, der in Fig. 2 nicht dargestellt ist, mit seiner Körperlängsachse 22 (vgl. Fig. 1) entlang der y-Achse der Fig. 2 ausgerichtet. Der Röntgenstrahl fällt senkrecht aus der z-Richtung kommend auf die Frontalebene 28 ein.

Die Einheitsvektoren in x-Richtung und z-Richtung spannen eine sog. Transversalebene 30.

Die Einheitsvektoren in y-Richtung und z-Richtung spannen eine sog. Sagittalebene.

Die einzelnen Komponenten $n_x$, $n_y$ und $n_z$ des Normalenvektors $\vec{n}$ lassen sich in Polarkoordinaten gemäß der radiologischen Bezeichnung von Murray in einem anatomischen Koordinatensystem, dessen Orientierung durch die sog. Beckeneingangsebene (APP-Konzept) gegeben ist, wie folgt ausdrücken:

$$\vec{n} \;=\; \begin{pmatrix} n_x \\ n_y \\ n_z \end{pmatrix} = \begin{pmatrix} \cos(RA)\sin(RI) \\ -\cos(RA)\cos(RI) \\ \sin(RA) \end{pmatrix} \qquad\qquad (Gl.\ 1)$$

wobei RA den radiologischen Anteversionswinkel und RI den radiologischen Inklinationswinkel bezeichnen. Nachfolgend werden Anteversionswinkel und Inklinationswinkel der Einfachheit halber auch lediglich als Anteversion bzw. Inklination bezeichnet.

Die Werte für die Inklination RI und die Anteversion RA können aus den Komponenten des Normalenvektors wie folgt berechnet werden:

$$\tan(RI) = \frac{n_x}{-n_y} \qquad und \qquad sin(RA) = n_z \qquad (Gl.\ 2a\ und\ Gl.\ 2b)$$

Wie bereits eingangs erwähnt, sollen die Inklination und die Anteversion korrekt bestimmt werden, und zwar in dem anatomischen Koordinatensystem des Patienten. Die bereits oben erwähnte Beckeneingangsebene ist in Fig. 3 dargestellt und ist mit dem Bezugszeichen 34 bezeichnet.

Fig. 3 zeigt eine schematische perspektivische Ansicht eines Beckenknochens 12 sowie ein Stück einer Wirbelsäule 20 (vgl. auch Fig. 1). In der Ansicht der Fig. 3 ist lediglich eine rechte Hüftpfanne 14a zu erkennen. Die Beckeneingangsebene 34 wird im anatomischen Bezugssystem als Referenzebene benutzt (Anterior - Pelvic - Plane (APP)-Konzept).

Ferner ist in der Fig. 3 eine Hüftpfannenöffnungsfläche 26 von einer gestrichelten Linie umrandet. Der Mittelpunkt dieser Fläche 26 dient als Ursprung für ein kartesisches Koordinatensystem, in dem der Normalenvektor n der Hüftpfanne 14a aufgespannt ist. Die xy-Ebene dieses kartesischen Koordinatensystems ist parallel zur Beckeneingangsebene 34 orientiert. Die Beckeneingangsebene 34 wird durch die beiden Spinae (spinae iliaca anterior superior, SIAS) am vorderen Beckenkamm sowie der Symphysenfuge gebildet.

Die Anteversion RA entspricht dem Winkel zwischen dem Pfannennormalenvektor $\vec{n}$ und der xy-Ebene (Polarwinkel). Die Inklination RI entspricht dem Drehwinkel des Normalenvektors um die z-Achse (Azimuthwinkel).

Bei einer idealen Patientenlagerung ist die Beckeneingangsebene 34 parallel zu einer Tischebene orientiert, auf der der Patient liegt. Ein solcher Tisch ist üblicherweise Teil einer Röntgenvorrichtung, wobei die Röntgenquelle senkrecht auf die Tischoberfläche einstrahlt und unterhalb der Tischoberfläche und parallel dazu bilderfassende Mittel, wie z.B. ein Röntgenfilm oder ein Röntgendetektor angeordnet ist. In diesem Fall beträgt die Beckenkippung dann 0°. Die Beckenkippung bzw. deren Winkel drückt eine Drehung um die x-Achse des kartesischen Koordinatensystems (der Hüftpfannen) aus. Im klinischen Alltag befindet sich der Patient während der Röntgenaufnahme jedoch in der Regel nicht in der optimalen Patientenlagerungsposition.

Die x-Achse des kartesischen Koordinatensystems der Fig. 3 ist durch eine (imaginäre) Verbindungslinie zwischen den Mittelpunkten der Hüftpfannenöffnungsflächen 26 definiert.

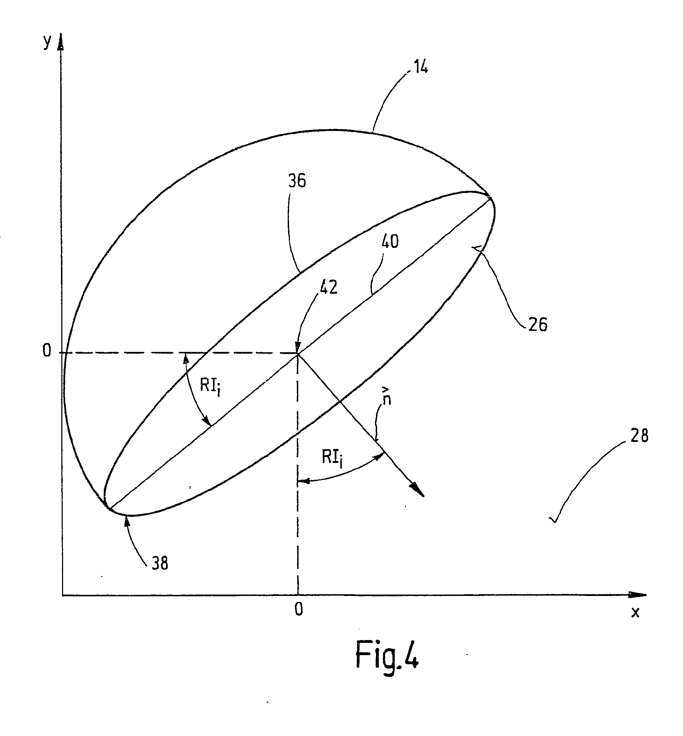

Die Hüftpfanne 14 kann durch eine Halbkugel idealisiert werden, wie es in Fig. 4 dargestellt ist.

Fig. 4 stellt einen Teilausschnitt aus einer schematisierten Röntgenaufnahme dar, wobei lediglich eine linke Hüftpfanne 14 dargestellt ist. Die in der Fig. 4 beispielhaft dargestellte Struktur erhält man z.B. mit der oben erwähnten a.p.-BÜS-Aufnahme, wobei die Zeichenebene der Fig. 4 mit der Ebene eines Röntgenfilms, der bei einer solchen Aufnahme verwendet wird, übereinstimmt. Diese Ebene entspricht der Frontalebene.

Vorausgesetzt, dass die Hüftpfanne 14 durch eine Halbkugel idealisiert wird, kann nun an Hand des Öffnungsrands 36 der Pfannenöffnungsfläche 26 die Orientierung der Hüftpfanne 14 aus dem entsprechenden Röntgenbild gewonnen werden.

Der Öffnungsrand 36 (der idealisierten halbkugelförmigen Hüftpfanne 14) wird dazu durch eine Ellipse genähert. Der Öffnungsrand 36 ist im Röntgenbild identifizierbar. Der Normalenvektor $\vec{n}$ der Hüftpfanne 14, oder präziser gesagt die Projektion des Normalenvektors, lässt sich mit Hilfe einer Hauptachse 40 der Ellipse 38 bestimmen. Um die Hauptachse 40 zu bestimmen, werden die größten und kleinsten Abstände des Pfannenöffnungsrands 36 zu einem Koordinatenursprung 42 ermittelt. Der Koordinatenursprung 42 entspricht dem Mittelpunkt der Pfannenöffnungsfläche 26, wie zuvor oben erläutert. Auf diese Weise lässt sich die Hauptachse 40 (per Auge, per Erkennungssoftware, etc.) aus den größten Abständen zum Koordinatenursprung 42 ermitteln.

Mit Hilfe der Hauptachse 42 lässt sich der (imaginäre, weil auf dem Röntgenbild nicht enthaltene) Normalenvektor $\vec{n}$ bestimmen. Der so bestimmte Normalenvektor $\vec{n}$ der Fig. 4 entspricht dem Normalenvektor $\vec{n}$ der Hüftpfanne 14 im radiologischen Bezugssystem (des Röntgenbilds). Die x-Achse der Fig. 4 wird dabei möglichst so gewählt, dass sie parallel zu der (nicht dargestellten) imaginären Verbindungslinie der Pfannenmittelpunkte verläuft, wie zuvor oben erläutert.

Der Inklinationswinkel $RI_i$ einer i-ten Röntgenaufnahme lässt sich somit numerisch bzw. geometrisch aus dem Winkel zwischen der Hauptachse 40 und einer Horizontalen erhalten.

Gemäß dem erfindungsgemäßen Verfahren werden zwei Bilder des Beckenknochens 12 erzeugt. Die Bilderzeugung kann unter Verwendung von Röntgentechniken, NMR-Techniken, etc. erfolgen.

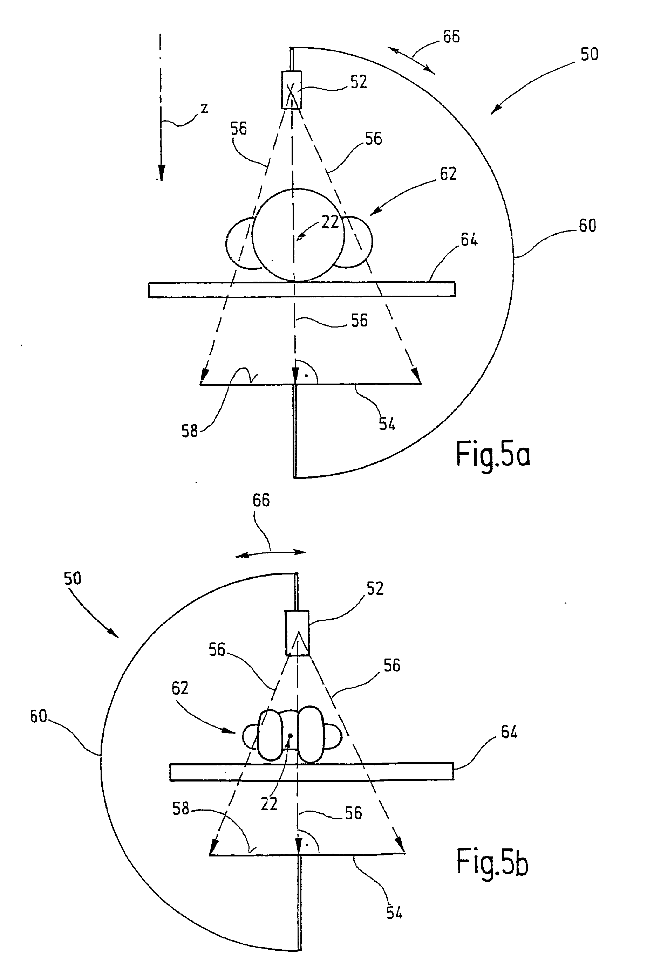

In den Fig. 5a und 5b ist jeweils ein Röntgengerät 50 dargestellt, das eine Röntgenquelle 52 sowie Röntgenerfassungsmittel 54, wie z.B. einen Röntgenfilm, aufweist, die von der Röntgenquelle emittierte Röntgenstrahlen 56 erfassen. Die Röntgenquelle 52 strahlt dabei senkrecht auf eine Erfassungsfläche 58 des Röntgenerfassungsmittels 54 ein. Die Röntgenquelle 52 und die Röntgenerfassungsmittel 54 sind beispielsweise mittels eines C-förmigen Rahmens 60 miteinander verbunden.

Ein Patient 62 liegt auf einem vorzugsweise horizontal ausgerichteten Tisch 54, wobei die Fläche des Tischs ebenfalls senkrecht zur Röntgenquelle 52 und somit parallel zur Fläche 58 der Röntgenerfassungsmittel 54 orientiert ist. Die Röntgenquelle 52 und die Röntgenerfassungsmittel 54 lassen sich mittels des C-förmigen Rahmens 60 vorzugsweise um die Körperlängsachse 22 des Patienten 62 drehen, wie es in den Fig. 5a und 5b durch Doppelpfeile angedeutet ist.

Die Fig. 5a zeigt das Röntgengerät 50, wobei man auf den Kopf des Patienten 62 blickt, und die Fig. 5b zeigt das Röntgengerät 50 aus der umgekehrten Richtung, d.h. man blickt auf die Füße des Patienten 62.

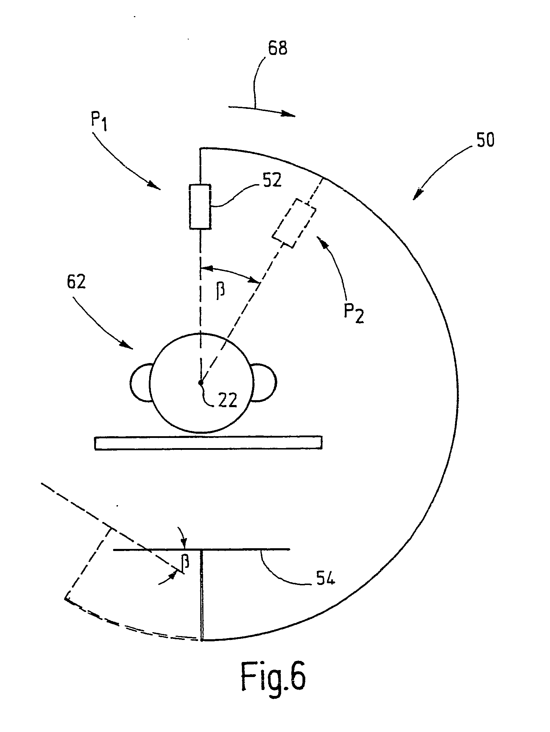

Fig. 6 zeigt zwei Positionen P1 und P2 des Röntgengeräts 50, bei denen jeweils eine Röntgenaufnahme gemacht wird. Die Position P2 des Röntgengeräts 50 ist durch eine gestrichelte Linie dargestellt.

In der Position P1 wird die a.p.-Röntgenaufnahme gewonnen. In der Position P2 wird eine zweite Röntgenaufnahme bzw. ein zweites Röntgenbild erzeugt, wobei das Röntgengerät 50 um einen beliebigen Bildrotationswinkel β, vorzugsweise um ca. 30° bis 50° und insbesondere um 40°, in Richtung des Pfeils 68 um die Körperlängsachse 22 des Patienten 62 herum gedreht wurde. Man beachte, dass der Patient 62 beim Erzeugen dieser beiden Aufnahmen nicht umgelagert werden muss. Auch die Lage und Ausrichtung der Röntgenquelle 52 zu den Röntgenerfassungsmitteln 54 bleibt erhalten.

Unter Verwendung von zwei solchen Röntgenaufnahmen kann jeweils die Inklination auf die oben beschriebene Art und Weise entweder manuell oder unter Einsatz von Computern (geometrisch) bestimmt werden. Aus den beiden so erzeugten Röntgenbildern lassen sich jeweils die Inklinationswinkel (im radiologischen Bezugssystem der Röntgenbilder) bestimmen. Ferner ist der Bildrotationswinkel β bekannt.

Zusätzlich kann aus den Röntgenbildern ein sog. Beckenrotationswinkel δ bestimmt werden.

Der Beckenrotationswinkel δ stellt den Winkel zwischen der (imaginären) Verbindungslinie der beiden auf dem Röntgenbild enthaltenen Hüftpfannenmittelpunkte zu einer Horizontalen des Röntgenbilds (bzw. der Frontalebene) dar. Klinisch wird diese Referenzlinie zur Bestimmung des Winkels δ üblicherweise aus den untersten Punkten der Ränder der beiden Schaufeln des Schambeins bestimmt. Der Beckenrotationswinkel δ drückt somit nichts anderes als eine Drehung des Beckens aus der oben beschriebenen optimalen Patientenlagerungsposition heraus aus um die z-Achse (vgl. Fig. 1, bei der die z-Achse aus der Figurenebene senkrecht herausragt) herum.

Ferner wird ein Beckenverkippungswinkel α z.B. unter Verwendung einer Beckenwaage bestimmt. Der Beckenverkippungswinkel α stellt die Verkippung der Beckeneingangsebene 34 (vgl. Fig. 3) um die x-Achse dar. Um die Frontalebene 28 parallel zur Beckeneingangsebene 34 zu orientieren, wird die Frontalebene 28 um α um die x-Achse, die der Verbindungslinie der Hüftpfannenmittelpunkte 42 entspricht, herum gedreht.

Der Beckenverkippungswinkel α kann beispielsweise während des Vorgangs des Erzeugens der beiden Bilder, wie in der Fig. 6 dargestellt ist, bestimmt werden. Dazu wird dem Patienten 62 eine (nicht dargestellte) Beckenwaage auf die beiden Spinae sowie auf die Symphysenfuge aufgelegt. Die Beckenwaage enthält eine Anzeige, aus der sich der Beckenverkippungswinkel α ablesen lässt.

Somit sind fünf Winkel bekannt. Die Inklinationswinkel $RI_i$ (i = 1, 2) des ersten und zweiten Bilds, der Bildrotationswinkel β, der Beckenrotationswinkel δ und der Beckenverkippungswinkel α.

Mit diesen Winkeln lässt sich die anatomische Inklination und die anatomische Anteversion, d.h. die Inklination und die Anteversion im anatomischen Bezugssystem, ermitteln, wie es nachfolgend erläutert wird.

Die Gleichung Gl. 2a gibt die radiologische Inklination RI an, die wiederum aus der Frontalebene 28 einer projizierten Linie des Normalenvektors $\vec{n}$ aus einem Röntgenbild gemessen werden kann.

Wie bereits erwähnt, ist das Becken 10 üblicherweise in Bezug auf die Frontalebene 28 um die Hüftrotationszentren verkippt, so dass die Beckeneingangsebene 34 nicht parallel zur Frontalebene 28 ist. Mathematisch entspricht dies einer (Ebenen)-Rotation um die x-Achse herum, und zwar um den Beckenrotationswinkel α. Ein ungestörter Normalenvektor $n_0$ einer Hüftpfanne ist deshalb in den Normalenvektor $n_1$ gemäß der folgenden Gleichung gedreht:

$$\vec{n}_0 \rightarrow \vec{n}_1 = A_x \cdot \vec{n}_0 = \begin{pmatrix} 1 & 0 & 0 \\ 0 & \cos(\alpha) & -\sin(\alpha) \\ 0 & \sin(\alpha) & \cos(\alpha) \end{pmatrix} \begin{pmatrix} n_x \\ n_y \\ n_z \end{pmatrix} = \begin{pmatrix} n_x \\ n_y \cos(\alpha) - n_z \sin(\alpha) \\ n_y \sin(\alpha) + n_z \cos(\alpha) \end{pmatrix} \quad \text{(Gl. 3)}$$

Aus den Komponenten des rotierten Normalenvektors $\vec{n}_1$ lässt sich ein gestörter Wert für den Inklinationswinkel RI1 berechnen:

$$\tan(RI1) = \frac{n_{1x}}{-n_{1y}} = \frac{n_x}{-(n_y \cos(\alpha) - n_z \sin(\alpha))} \quad \text{(Gl. 4a)}$$

Diese Gleichung lässt sich umschreiben zu

$$\cot(RI1) \quad = \quad \frac{1}{\tan(RI1)} = \frac{-n_y}{n_z}\cos(\alpha) + \frac{-n_z}{n_x}\sin(\alpha)$$

$$= \quad \cot(RI)\cos(\alpha) + \tan(RA)\frac{1}{\sin(RI)}\sin(\alpha) \qquad (Gl. \ 4b)$$

Da die Patientenausrichtung üblicherweise auch bezüglich der Körperlängsachse nicht ideal ist, ist das Becken nicht nur lediglich um die x-Achse, sondern auch um die z-Achse zu drehen. Somit kann die Rotation des Beckens in der Frontalebene 28 durch eine nachfolgende Rotation um die z-Ebene mit dem Rotationswinkel δ wie folgt ausgedrückt werden:

$$\vec{n}_1 \ \to \ \vec{n}_2 = A_2 \ \cdot \ \vec{n}_1 \ = \ \begin{pmatrix} \cos(\delta) & -\sin(\delta) & 0 \\ \sin(\delta) & \cos(\delta) & 0 \\ 0 & 0 & 1 \end{pmatrix} \begin{pmatrix} n_{1x} \\ n_{1y} \\ n_{1z} \end{pmatrix} \qquad (Gl. \ 5a)$$

Unter Berücksichtigung der Gleichung 3 erhält man somit:

$$\begin{pmatrix} n_{2x} \\ n_{2y} \\ n_{2z} \end{pmatrix} = \begin{pmatrix} n_x\cos(\delta) - (n_y\cos(\alpha) - n_z\sin(\alpha))\sin(\delta) \\ n_x\sin(\delta) + (n_y\cos(\alpha) - n_z\sin(\alpha))\cos(\delta) \\ n_y\sin(\alpha) + n_z\cos(\alpha) \end{pmatrix}$$

$$(Gl. \ 5b)$$

Der Wert für die (erneut) gestörte Inklination RI2 lässt sich somit ermitteln durch:

$$\tan(RI2) \quad = \quad \frac{n2_x}{-n2_y} = \frac{n_x\cos(\delta) - (n_y\cos(\alpha) - n_z\sin(\alpha))\sin(\delta)}{-(n_x\sin(\delta) + (n_y\cos(\alpha) - n_z\sin(\alpha))\cos(\delta))} \qquad (Gl. \ 6)$$

wobei die Inklination RI2 dem gemessenen bzw. zu messenden Inklinationswinkel des ersten Bildes entspricht.
Die Gleichung 6 ergibt eine lineare Gleichung für die Komponenten des ungestörten Normalenvektors $n_0$.

$$0 = n_x\cos(\delta) \ - \ (n_y\cos(\alpha) \ - \ n_z\sin(\alpha))\sin(\delta) \ + \ \tan(RI2)((n_x\sin(\delta) \ + \ (n_y\cos(\alpha)$$

$$- \ n_z\sin(\alpha))\cos(\delta)) \qquad\qquad (Gl. \ 7)$$

Das zweite Bild, welches beispielsweise aus der Position P2 der Fig. 6 aufgenommen wird, wird in den nachfolgenden Gleichungen benutzt werden, um die Information über den zweiten Inklinationswinkel des zweiten Bildes zu nutzen. Bei der Aufnahme des zweiten Bildes wird das Röntgengerät z.B. um die Körperlängsachse 22 des Patienten 62 um den Bildrotationswinkel β gedreht.

Mathematisch lässt sich diese zusätzliche Drehung um die y-Achse in der Frontalebene wie folgt ausdrücken, wobei die Gleichung 5a zu berücksichtigen ist:

$$\vec{n}_2 \rightarrow \vec{n}_3 = A_y \cdot \vec{n}_2 = \begin{pmatrix} \cos(\beta) & 0 & \sin(\beta) \\ 0 & 1 & 0 \\ -\sin(\beta) & 0 & \cos(\beta) \end{pmatrix} \begin{pmatrix} n_{2x} \\ n_{2y} \\ n_{2z} \end{pmatrix} \qquad \text{(Gl. 8a)}$$

$$\begin{pmatrix} n_{3x} \\ n_{3y} \\ n_{3z} \end{pmatrix} = \begin{pmatrix} \left[n_x \cos(\delta) - \left(n_y \cos(\alpha) - n_z \sin(\alpha)\right)\sin(\delta)\right]\cos(\beta) + \left[n_y \sin(\alpha) + n_z \cos(\alpha)\right]\sin(\beta) \\ n_x \sin(\delta) + \left(n_y \cos(\alpha) - n_z \sin(\alpha)\right)\cos(\delta) \\ -\left[n_x \cos(\delta) - \left(n_y \cos(\alpha) - n_z \sin(\alpha)\right)\sin(\delta)\right]\sin(\beta) + \left[n_y \sin(\alpha) + n_z \cos(\alpha)\right]\cos(\beta) \end{pmatrix}$$

(Gl. 8b)

Wie zuvor erhält man für den projizierten Inklinationswinkel RI3, welcher dem im zweiten Bild gemessenen Inklinationswinkel entspricht, die folgende Gleichung:

$$\tan(RI3) = \frac{\left[n_x \cos(\delta) - \left(n_y \cos(\alpha) - n_z \sin(\alpha)\right)\sin(\delta)\right]\cos(\beta) + \left[n_y \sin(\alpha) + n_z \cos(\alpha)\right]\sin(\beta)}{-\left(n_x \sin(\delta) + \left(n_y \cos(\alpha) - n_z \sin(\alpha)\right)\cos(\delta)\right)}$$

$$0 = \left[n_x \cos(\delta) - \left(n_y \cos(\alpha) - n_z \sin(\alpha)\right)\sin(\delta)\right]\cos(\beta) + \left[n_y \sin(\alpha) + n_z \cos(\alpha)\right]\sin(\beta)$$
$$+ \tan(RI3)\left(n_x \sin(\delta) + \left(n_y \cos(\alpha) - n_z \sin(\alpha)\right)\cos(\delta)\right)$$

(Gl. 9)

Die Gleichung 9 stellt ebenfalls eine lineare Gleichung dar, die die unbekannten Komponenten $n_x$, $n_y$, und $n_z$ des Normalenvektors $n_0$ enthält.

Somit liegen zwei Gleichungen (Gleichung 7 und 9) für die drei unbekannten Komponenten des Normalenvektors vor. Berücksichtigt man nun zusätzlich die Gleichung 1, d.h. dass der Normalenvektor sich auch in Polarkoordinaten darstellen lässt, so erkennt man, dass zwei lineare Gleichungen mit insgesamt zwei unbekannten Größen vorliegen. Die unbekannten Größen sind der Azimuth und der Polarwinkel. Der Azimuth und der Polarwinkel wiederum entsprechen der Inklination und der Anteversion im anatomischen Bezugssystem. Diese beiden Winkel erhält man wie folgt:

Ausgehend von den Gleichungen 7 und 9 kann man ein lineares Gleichungssystem erstellen, indem man Gleichung 7 überführt in Gleichung 7c:

$$0 = n_x \cos(\delta) - n_y \cos(\alpha)\sin(\delta) + n_z \sin(\alpha)\sin(\delta)$$
$$+ n_x \sin(\delta)\tan(RI2) + n_y \cos(\alpha)\cos(\delta)\tan(RI2) - n_z \sin(\alpha)\cos(\delta)\tan(RI2)$$

(Gl. A7a)

$$0 = n_x \cos(\delta) + n_x \sin(\delta)\tan(RI2) - n_y \cos(\alpha)\sin(\delta) + n_y \cos(\alpha)\cos(\delta)\tan(RI2)$$
$$+ n_z \sin(\alpha)\sin(\delta) - n_z \sin(\alpha)\cos(\delta)\tan(RI2)$$

(Gl. A7b)

$$0 = (\cos(\delta) + \sin(\delta)\tan(RI2)) \cdot n_x + (-\cos(\alpha)\sin(\delta) + \cos(\alpha)\cos(\delta)\tan(RI2)) \cdot n_y$$
$$+ (\sin(\alpha)\sin(\delta) - \sin(\alpha)\cos(\delta)\tan(RI2)) \cdot n_z$$

(Gl. A7c)

Ähnliches gilt für die Gleichung A9

$$0 = n_x \cos(\delta)\cos(\beta) - n_y \cos(\alpha)\sin(\delta)\cos(\beta) + n_z \sin(\alpha)\sin(\delta)\cos(\beta)$$
$$+ n_y \sin(\alpha)\sin(\beta) + n_z \cos(\alpha)\sin(\beta)$$
$$+ n_x \sin(\delta)\tan(RI3) + n_y \cos(\alpha)\cos(\delta)\tan(RI3) - n_z \sin(\alpha)\cos(\delta)\tan(RI3)$$

(Gl. A9a)

$$0 = (\cos(\delta)\cos(\beta) + \sin(\delta)\tan(RI3)) \cdot n_x$$
$$+ (-\cos(\alpha)\sin(\delta)\cos(\beta) + \sin(\alpha)\sin(\beta) + \cos(\alpha)\cos(\delta)\tan(RI3)) \cdot n_y$$
$$+ (\sin(\alpha)\sin(\delta)\cos(\beta) + \cos(\alpha)\sin(\beta) - \sin(\alpha)\cos(\delta)\tan(RI3)) \cdot n_z$$

(Gl. A9b)

Gleichung 7c und 9b repräsentieren ein System linearer Gleichungen für die unbekannten Werte der Komponenten $n_x$, $n_y$ und $n_z$ des Normalenvektors $n_0$. Dieses Gleichungssystem lässt sich wie folgt schreiben:

$$\begin{pmatrix} 0 \\ 0 \\ 0 \end{pmatrix} = \begin{pmatrix} a11 & a12 & a13 \\ a21 & a22 & a23 \\ 0 & 0 & 0 \end{pmatrix} \begin{pmatrix} n_x \\ n_y \\ n_z \end{pmatrix} = A \cdot \vec{n}_0$$

$$(Gl. \ A10)$$

Die dritte Zeile der Matrix A wurde willkürlich hinzugefügt und stellt nichts anderes als die Gleichung 0 = 0 dar. Die ersten beiden Koeffizienten der Matrix $A_{ij}$ lauten beispielsweise

$$a11 = \cos(\delta) + \sin(\delta) \ \tan(RI2)$$

$$a12 = - \cos(\alpha) \ \sin(\delta) + \cos(\alpha) \ \cos(\delta) \ \tan(RI2)$$

Die Gleichung 10 stellt ein lineares Gleichungssystem mit zwei Gleichungen und drei Unbekannten ($n_x$, $n_y$, $n_z$) dar. Eine solche Gleichung hat neben der trivialen Lösung $(0,0,0)^T$ eine weitere Lösung, nämlich einen Vektor, der die Richtung einer Gerade definiert.

Fasst man die Gleichung 10 als lineare Abbildung auf, so gibt es neben dem trivialen Lösungsvektor $(0,0,0)^T$ einen Vektor $(nx, ny, nz)^T$, der auf den Null-Vektor abgebildet wird, und zwar mit dem Eigenwert Null.

Da der Normalenvektor der Hüftpfanne die Orientierung der Hüftpfanne eindeutig charakterisiert, lässt sich an Hand der messbaren Hauptachsen 40 (vgl. Fig. 4) der ellipsenförmigen Umrisslinie 36 die Projektion des Normalenvektors in der Frontalebene und somit der Inklinationswinkel bestimmen. Dies bedeutet, dass man durch Messung der Hauptachse eine identische Information über die Hüftpfannenausrichtung erhält wie durch die Projektion des Normalenvektors der Hüftpfanne.

Eine exemplarische Ausführungsform des Verfahrens gemäß der Erfindung lässt sich wie folgt zusammenfassen: Aus einer ersten normalen anterior-posterior-Röntgenaufnahme wird die Hauptachse der interessierenden Hüftpfanne bestimmt; ferner wird z.B. auf dem ersten Bild die Orientierung des Beckens (d.h. der Winkel $\delta$) in der Frontalebene bestimmt; ein zweites Röntgenbild wird auf Grund einer definierten Rotation des Röntgengeräts um die Längsachse des Patienten erhalten, ohne die Position des Patienten zu verändern; an Hand des zweiten Bilds lässt sich wiederum die projizierte Hauptachse der Hüftpfanne feststellen, und somit auch ein zweiter Inklinationswinkel; und durch eine Zusatzmessung wird die Beckenneigung $\alpha$ beispielsweise mittels einer Beckenwaage bestimmt. Diese fünf Winkelwerte werden in die zuvor erläuterten Gleichungen eingesetzt, um diese nach der Inklination und der Anteversion im anatomischen Bezugssystem aufzulösen.

Da alle Messungen mit Messfehlern behaftet sind, wurden Simulationsrechnungen vorgenommen, die den Einfluss von Abweichungen der zuvor erwähnten Winkel berücksichtigen.

Die nachfolgende Tabelle 1

| Parameter | Fehler RA [Grad/Grad] | Fehler RI [Grad/Grad] |
|---|---|---|
| RI2 | 1,8 | 1,22 |
| RI3 | 2,3 | 0,3 |
| $\alpha$ | 0,71 | 0,19 |
| $\beta$ | 0,244 | 0,04 |
| $\delta$ | 0,123 | 0,95 |

(fortgesetzt)

| Parameter | Fehler RA [Grad/Grad] | Fehler RI [Grad/Grad] |
|---|---|---|
|  |  |  |

zeigt den Einfluss von Messfehlern, wobei die fünf Winkelparameter um jeweils $\pm$ 2 Grad unabhängig voneinander variiert wurden, während die restlichen konstant gehalten wurden.

Aus Tabelle 1 erkennt man, dass der Wert für RI3 von allen Parametern der kritischste Wert ist.

Jedoch erkennt man aus Tabelle 1 auch, dass sich die Fehler in einem akzeptablen Bereich bewegen, verglichen mit den Methoden gemäß dem Stand der Technik (Sven-Johansson, Visser-Technik), die die Beckenkippung gar nicht erst berücksichtigen.

Es versteht sich, dass zur Erzeugung geeigneter Bilder nicht ausschließlich Röntgenstrahlen verwendet werden können. Im Stand der Technik sind andere bildgebende Verfahren bekannt, die hier ebenfalls angewendet werden könnten. Ferner ist die Verwendung eines sog. C-Bogens nicht zwingend erforderlich. Andere Vorrichtungen können benutzt werden, bei denen die bilderzeugenden Mittel und die bilderfassenden Mittel fest zueinander angeordnet sind. Ferner muss die Körperlängsachse des Patienten nicht mit der Längsachse eines Tischs übereinstimmen, auf dem der Patient während der Aufnahme der Bilder liegt. Eine "Fehlausrichtung" des Patienten wird auf Grund des Beckenrotationswinkels $\delta$ berücksichtigt.

**Patentansprüche**

1. Verfahren zum Bestimmen des Inklinationswinkels (RI) und des Anteversionswinkels (RA) einer Hüftpfanne (14a; 14b) eines Patienten in einem anatomischen Bezugssystem, dessen Orientierung durch den Beckenknochen (12) des Patienten bestimmt ist, unter Berücksichtigung einer Beckenverkippung, wobei das Verfahren die folgenden Schritte aufweist:

   Bereitstellen eines ersten Bilds des Beckenknochens (12), wobei das erste Bild eine Frontalebene (XY) mit dem Beckenknochen (12) zeigt;
   Bestimmen eines ersten Inklinationswinkels (RI2) der Hüftpfanne (14a; 14b) unter Verwendung des ersten Bilds;
   Bestimmen eines Beckenrotationswinkels ($\delta$), der den Rotationswinkel des Beckenknochens (12) um die Normale (Z) der Frontalebene (XY) relativ zu einer optimalen Patientenlagerungsposition angibt;
   Bereitstellen eines zweiten Bilds des Beckenknochens (12), wobei die Ebene des zweiten Bilds um einen Bildrotationswinkel ($\beta$) relativ zu der Frontalebene um eine gemeinsame Schnittlinie (Y; 22) der beiden Ebenen herum, vorzugsweise um die Körperlängsachse (22) des Patienten herum, rotiert ist;
   Bestimmen eines zweiten Inklinationswinkels (RI3) der Hüftpfanne (14a; 14b) unter Verwendung des zweiten Bilds;
   Bestimmen eines Beckenverkippungswinkels ($\alpha$), der eine Rotation der Frontalebene des ersten Bildes relativ zu einer Beckeneingangsebene in der optimalen Patientenlagerungsposition repräsentiert;
   Aufstellen eines linearen Gleichungssystems mit mindestens zwei linearen Gleichungen in Abhängigkeit von den zuvor bestimmten Winkeln ($\alpha$, $\beta$, $\delta$, RI2, RI3);
   Ausdrücken des Normalenvektors $n_0$ in Polarkoordinaten des anatomischen Bezugssystems;
   Auflösen der zumindest zwei linearen Gleichungen nach Azimuth und Polarwinkel des Normalenvektors $n_0$, um so die Inklination (RI) und die Anteversion (RA) zu bestimmen.

2. Verfahren nach Anspruch 1, wobei das Gleichungssystem einer linearen Abbildung A entspricht, die den Normalenvektor $n_0$ der Hüftpfannenöffnungsebene (26) auf den Null-Vektor des anatomischen Bezugssystems abbildet, und zwar in der Form:

$$\begin{pmatrix} 0 \\ 0 \\ 0 \end{pmatrix} = \begin{pmatrix} a11 & a12 & a13 \\ a21 & a22 & a23 \\ 0 & 0 & 0 \end{pmatrix} \begin{pmatrix} n_x \\ n_y \\ n_z \end{pmatrix} = A \cdot \vec{n}_0$$

wobei $n_0$ den Normalenvektor der Hüftpfannenöffnungsebene (26) repräsentiert und die Matrixelemente $a_{ij}$ durch die zuvor bestimmten Winkel bestimmt sind.

3. Verfahren nach Anspruch 2, wobei alternativ zum Auflösen der zumindest zwei linearen Gleichungen nach Azimuth und Polarwinkel des Normalenvektors $n_0$, der Azimuth und der Polarwinkel des Normalenvektors $n_0$ durch Bestimmen des Eigenvektors der Abbildung A beim Eigenwert Null bestimmt wird.

4. Verfahren nach einem der vorhergehenden Ansprüche, wobei eine erste Gleichung der linearen Gleichungen, eine Drehungen der Hüftpfanne (14a; 14b) um den Beckenverkipplungswinkel (α) und um den Beckenrotationswinkel (δ) zum Darstellen des ersten Inklinationswinkels (RI2) repräsentiert, und eine zweite Gleichung der linearen Gleichungen eine weitere Drehung der ersten Gleichung um den Bilddrehwinkel (β) zum Darstellen des zweiten Inklinationswinkels (RI3) repräsentiert.

5. Verfahren nach einem der vorhergehenden Ansprüche, wobei die optimale Patientenlagerungsposition **dadurch** definiert ist, dass die Beckeneingangsebene (34) parallel zur Frontalebene (28) orientiert ist und dass eine Verbindungslinie der Hüftpfannenmittelpunkte (42) parallel zu einem der Einheitsvektoren (x) orientiert ist, der die Frontalebene (28) aufspannt, wobei der andere Einheitsvektor (y) parallel zur Körperlängsachse (22) des Patienten (62) orientiert ist.

6. Verfahren nach einem der vorhergehenden Ansprüche, wobei das anatomische Bezugssystem durch die Beckeneingangsebene (34) definiert ist, welche durch die Spinae (SIAS) am vorderen Beckenkamm sowie die Symphysenfuge (SP) des Beckenknochens (12) definiert ist.

7. Verfahren nach einem der vorhergehenden Ansprüche, wobei ein bildgebendes System (50) zur Erzeugung des zweiten Bildes um die Körperlängsachse (22) des Patienten rotiert wird.

8. Verfahren nach einem der vorhergehenden Ansprüche, wobei der erste und zweite Inklinationswinkel (RI2, RI3) jeweils mittels eines Normalenvektors (n) bestimmt werden, der jeweils senkrecht auf einer Öffnungsfläche (26) der Hüftpfanne (14a; 14b) steht, wie sie im ersten Bild bzw. zweiten Bild abgebildet ist.

9. Verfahren nach Anspruch 8, wobei der jeweilige Normalenvektor (n) derart bestimmt wird, dass die Hüftpfanne (14a; 14b) durch eine offene Halbkugel genähert wird, wobei ein Öffnungsrand (36) der jeweiligen Halbkugel durch eine Ellipse genähert wird, deren Hauptachsen (40) ermittelt werden, zu denen der jeweilige Normalenvektor orthogonal (n) orientiert ist.

10. Verfahren nach Anspruch 8 oder 9, wobei der erste und zweite Inklinationswinkel (RI2, RI3) geometrisch anhand des jeweiligen Winkels zwischen dem Normalenvektor (n) und der Horizontalen (x) der Frontalebene (28) bestimmt wird.

11. Verfahren nach einem der vorhergehenden Ansprüche, wobei der Ursprung (42) der Frontalebene (28) in den Mittelpunkt der Hüftpfanne (14a; 14b) gelegt wird.

12. Verfahren nach einem der vorhergehenden Ansprüche, wobei die Bilder in Form von jeweiligen Datensätzen bereitgestellt werden.

13. Verfahren nach einem der vorhergehenden Ansprüche, wobei die Bilder mittels Röntgenaufnahmen oder NMR bereitgestellt werden.

14. Verfahren nach einem der vorhergehenden Ansprüche, wobei der Beckenrotationswinkel (δ) geometrisch als Winkel zwischen einer Horizontalen (y) der Frontalebene (28) und entweder einer Verbindungslinie zwischen den Hüftpfannenmittelpunkten (42) oder einer Verbindungslinie zwischen untersten Randpunkten des Schambeins bestimmt wird.

15. Verfahren nach einem der vorhergehenden Ansprüche, wobei der Beckenverkippungswinkel (α) unter Verwendung einer Beckenwaage bestimmt wird.

16. Verfahren nach einem der vorhergehenden Ansprüche, wobei die Bilder unter Verwendung eines bildgebenden Systems (50) bereitgestellt werden, das eine Strahlungsquelle (52) und eine Erfassungseinrichtung (54) umfasst, die starr zueinander angeordnet sind und insbesondere um eine die Körperlängsachse (22) des Patienten (62) drehbar sind.

**17.** Verfahren nach Anspruch 16, wobei das bildgebende System (50) einen C-Bogen (60) umfasst.

**Claims**

**1.** A method for determining in an anatomical reference system the inclination angle (RI) and the anteversion angle (RA) of a patient's acetabulum (14a; 14b), the orientation of which is determined by the patient's pelvic bone (12), wherein tilt of the pelvis is taken into consideration, the method comprising the steps of:

providing a first image of the pelvic bone (12) wherein the first image represents a frontal plane (XY) having the pelvic bone (12);
determining a first inclination angle (RI2) of the acetabulum (14a; 14b) by using the first image;
determining a pelvis rotation angle ($\delta$) which indicates the rotation angle of the pelvic bone (12) about the normal (Z) of the frontal plane (XY) relative to an optimum patient location position;
providing a second image of the pelvic bone (12) wherein the second image plane is rotated by an image rotation angle ($\beta$) relative to the frontal plane about a common section line (Y; 22) of the two planes, preferably about the patient's longitudinal body axis (22);
determining a second inclination angle (RI3) of the acetabulum (14a; 14b) by using the second image;
determining a pelvis-tilt angle ($\alpha$) which represents a rotation of the frontal plane of the first image relative to an anterior-pelvic plane in the optimum patient location position;
setting up a linear equation system having at least two linear equations as a function of the previously determined angles ($\alpha$, $\beta$, $\delta$, RI2, RI3);
expressing the normal vector $n_0$ in polar coordinates of the anatomical reference system; and
solving the at least two linear equations for the azimuth and polar angle of the normal vector $n_0$ for determining the inclination (RI) and the anteversion (RA).

**2.** The method of claim 1, wherein the equation system corresponds to a linear mapping A which maps the normal vector $n_0$ of the acetabulum opening plane (26) onto the zero vector of the anatomical reference system, to be precise in the form:

$$\begin{pmatrix} 0 \\ 0 \\ 0 \end{pmatrix} = \begin{pmatrix} a11 & a12 & a13 \\ a21 & a22 & a23 \\ 0 & 0 & 0 \end{pmatrix} \begin{pmatrix} n_x \\ n_y \\ n_z \end{pmatrix} = A \cdot \vec{n}_0$$

wherein $n_0$ represents the normal vector of the acetabulum opening plane (26), and the matrix elements $a_{ij}$ are determined by the previously determined angles.

**3.** The method of claim 2, wherein, as an alternative to the solving of the at least two linear equations for the azimuth and polar angle of the normal vector $n_0$, the azimuth and the polar angle of the normal vector $n_0$ are determined by determining the Eigen vector of the mapping A for the Eigen value of zero.

**4.** The method of any one of the preceding claims, wherein a first equation of the linear equations represents a rotation of the acetabulum (14a; 14b) about the pelvis-tilt angle ($\alpha$) and about the pelvis-rotation angle ($\delta$) in order to represent the first inclination angle (RI2), and wherein a second equation of the linear equations represents another rotation of the first equation about the image rotation angle ($\beta$) in order to represent the second inclination angle (RI3).

**5.** The method of any one of the preceding claims, wherein the optimum patient location position is defined such that the anterior-pelvic plane (34) is oriented parallel to the frontal plane (28) and a line connecting the acetabulum center points (42) is oriented parallel to one of the unit vectors (x) which spans the frontal plane (28), wherein the other unit vector (y) is oriented parallel to the longitudinal axis (22) of the patient's body (62).

**6.** The method of any one of the preceding claims, wherein the anatomical reference system is defined by the anterior-pelvic plane (34) which is defined by the spinae (SIAS) at the front iliac crest as well as the symphysis (SP) of the pelvic bone (12).

7. The method of any one of the preceding claims, wherein an imaging system (50) is rotated about the body longitudinal axis (22) of the patient in order to produce the second image.

8. The method of any one of the preceding claims, wherein the first and second inclination angles (RI2, RI3) are each determined by means of a normal vector (n) which is respectively perpendicular to an opening surface (26) of the acetabulum (14a; 14b) as depicted in the first image and the second image, respectively.

9. The method of claim 8, wherein the respective normal vector (n) is determined such that the acetabulum (14a; 14b) is approximated by an open hemisphere, wherein an opening edge (36) of the respective hemisphere is approximated by an ellipse, major axes (40) of which are determined, the respective normal vector being oriented orthogonally (n) thereto.

10. The method of claim 8 or 9, wherein the first and second inclination angles (RI2, RI3) are determined geometrically on the basis of the respective angle between the normal vector (n) and the horizontal (x) of the frontal plane (28).

11. The method of any one of the preceding claims, wherein the origin (42) of the frontal plane (28) is into the center point of the acetabulum (14a; 14b).

12. The method of any one of the preceding claims, wherein the images are provided in terms of respective data sets.

13. The method of any one of the preceding claims, wherein the images are provided by means of X-ray images NMR.

14. The method of any one of the preceding claims, wherein the pelvis rotation angle ($\delta$) is determined geometrically as the angle between a horizontal (y) of the frontal plane (28) and one of a connecting line between the acetabulum center points (42) and a connecting line between the lowest edge points of the pubic bone.

15. The method of any one of the preceding claims, wherein the pelvis tilt angle ($\alpha$) is determined by using a pelvis balance.

16. The method of any one of the preceding claims, wherein the images are provided by using an image-providing system (50) which has a radiation source (52) and a detection device (54), which are arranged rigidly to each other and, in particular, can be rotated about the longitudinal axis (22) of the patient's body (62).

17. The method of claim 16, wherein the image-providing system (50) includes a C-arc (60).


**Revendications**

1. Procédé pour déterminer l'angle d'inclinaison (RI) et l'angle d'antéversion (RA) d'un cotyle (14a ; 14b) d'un patient dans un système de référence anatomique dont l'orientation est déterminée par l'os du bassin (12) du patient, en tenant compte d'un défaut d'alignement angulaire du bassin, le procédé présentant les étapes suivantes :

Fourniture d'une première image de l'os du bassin (12), la première image montrant un plan frontal (XY) avec l'os du bassin (12) ;
Détermination d'un premier angle d'inclinaison (RI2) du cotyle (14a ; 14b) en utilisant la première image ;
Détermination d'un angle de rotation du bassin ($\delta$) qui indique l'angle de rotation de l'os du bassin (12) autour de la normale (Z) du plan frontal (XY) par rapport à une position allongée optimale du patient ;
Fourniture d'une deuxième image de l'os du bassin (12), le plan de la deuxième image étant tournée d'un angle de rotation d'image ($\beta$) par rapport au plan frontal autour d'une ligne d'intersection commune (Y ; 22) des deux plans, de préférence autour de l'axe longitudinal du corps (22) du patient ;
Détermination d'un deuxième angle d'inclinaison (RI3) du cotyle (14a ; 14b) en utilisant la deuxième image ;
Fourniture d'un angle de défaut d'alignement angulaire du bassin ($\alpha$) qui représente une rotation du plan frontal de la première image par rapport à un plan d'entrée du bassin dans la position allongée optimale du patient ;
Établissement d'un système d'équations linéaire comprenant au moins deux équations linéaires en fonction des angles déterminés précédemment ($\alpha$, $\beta$, $\delta$, RI2, RI3) ;
Expression du vecteur normal $n_0$ en coordonnées polaires du système de référence anatomique ;
Résolution des au moins deux équations linéaires d'après l'azimut et l'angle polaire du vecteur normal $n_0$ afin

de déterminer ainsi l'inclinaison (RI) et l'antéversion (RA).

2. Procédé selon la revendication 1, le système d'équation correspondant à une représentation linéaire A qui représente le vecteur normal $n_0$ du plan d'ouverture du cotyle (26) sur le vecteur nul du système de référence anatomique, et ce sous la forme :

$$\begin{pmatrix} \bar{0} \\ 0 \\ 0 \end{pmatrix} = \begin{pmatrix} a11 & a12 & a13 \\ a21 & a22 & a23 \\ 0 & 0 & 0 \end{pmatrix} \begin{pmatrix} n_x \\ n_y \\ n_z \end{pmatrix} = A.\overline{n_0}$$

$n_0$ représentant ici le vecteur normal du plan du cotyle (26) et l'élément de matrice $a_{ij}$ étant défini par l'angle déterminé précédemment.

3. Procédé selon la revendication 2, l'azimut et l'angle polaire du vecteur normal $n_0$ étant déterminés en déterminant le vecteur propre de la représentation A avec une valeur propre nulle en variante de la résolution des au moins deux équations linéaires d'après l'azimut et l'angle polaire du vecteur normal $n_0$.

4. Procédé selon l'une des revendications précédentes, une première équation des équations linéaires représentant une rotation du cotyle (14a ; 14b) autour de l'angle de défaut d'alignement angulaire du bassin ($\alpha$) et autour de l'angle de rotation du bassin ($\delta$) en vue de représenter le premier angle d'inclinaison (RI2) et une deuxième équation des équations linéaires représentant une rotation supplémentaire de la première équation autour de l'angle de rotation d'image ($\beta$) en vue de représenter le deuxième angle d'inclinaison (RI3).

5. Procédé selon l'une des revendications précédentes, la position allongée optimale du patient étant définie en ce que le plan d'entrée du bassin (34) est orienté parallèlement au plan frontal (28) et qu'une ligne de liaison des points centraux du cotyle (42) est orientée parallèlement à un vecteur unitaire (x) qui couvre le plan frontal (28), l'autre vecteur unitaire (y) étant orienté parallèlement à l'axe longitudinal du corps (22) du patient (62).

6. Procédé selon l'une des revendications précédentes, le système de référence anatomique étant défini par le plan d'entrée du bassin (34), lequel est défini par l'épine dorsale (SIAS) sur la crête avant du bassin ainsi que par la symphyse pubienne (SP) de l'os du bassin (12).

7. Procédé selon l'une des revendications précédentes, un système d'imagerie (50) destiné à générer la deuxième image exécutant une rotation autour de l'axe longitudinal du corps (22) du patient.

8. Procédé selon l'une des revendications précédentes, le premier et le deuxième angles d'inclinaison (RI2, RI3) étant respectivement déterminés au moyen d'un vecteur normal (n) qui est à chaque fois perpendiculaire à une surface d'ouverture (26) du cotyle (14a ; 14b), telle qu'elle est illustrée dans la première image ou dans la deuxième image.

9. Procédé selon la revendication 8, le vecteur normal (n) correspondant étant déterminé de telle sorte que le cotyle (14a ; 14b) est défini approximativement par une demi-sphère ouverte, un bord d'ouverture (36) de la demi-sphère correspondante étant défini approximativement par une ellipse de laquelle sont déterminés les axes principaux (40) par rapport auxquels le vecteur normal (n) correspondant présente une orientation orthogonale.

10. Procédé selon la revendication 8 ou 9, le premier et le deuxième angles d'inclinaison (RI2, RI3) étant déterminés de manière géométrique au moyen de l'angle correspondant entre le vecteur normal (n) et l'horizontale (x) du plan frontal (28).

11. Procédé selon l'une des revendications précédentes, l'origine (42) du plan frontal (28) étant placée au point central du cotyle (14a ; 14b).

12. Procédé selon l'une des revendications précédentes, les images étant fournies sous la forme de jeux de données correspondants.

13. Procédé selon l'une des revendications précédentes, les images étant fournies au moyen de radiographies ou de RMN.

14. Procédé selon l'une des revendications précédentes, l'angle de rotation du bassin ($\delta$) étant déterminé de manière géométrique sous la forme de l'angle entre un horizontale (y) du plan frontal (28) et soit une ligne de liaison entre le point central du cotyle (42), soit une ligne de liaison entre le point de bord le plus bas du pubis.

**15.** Procédé selon l'une des revendications précédentes, l'angle de défaut d'alignement angulaire du bassin ($\alpha$) étant déterminé en utilisant une balance à bassin.

**16.** Procédé selon l'une des revendications précédentes, les images étant fournies en utilisant un système d'imagerie (50) qui comprend une source de rayonnement (52) et un dispositif d'acquisition (54), lesquels sont disposés de manière rigide l'un par rapport à l'autre et peuvent notamment tourner autour de l'un des axes longitudinaux du corps (22) du patient (62).

**17.** Procédé selon la revendication 16, le système d'imagerie (50) comprenant un étrier en C (60).

Fig.1

Fig.2

Fig 3

Fig.4

Fig.5a

Fig.5b

Fig.6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **von D. W. Murray.** The Definition and Measurement of Acetabular Orientation. *THE JOURNAL OF BONE AND JOINT SURGERY,* Marz 1993, vol. 75-B (2), 228-232

- **Johansson, S.** Zur Technik der Osteosynthese der Fratt. coli femoris. *Zbl. Chir.,* 1932, vol. 59, 2019-2022
- **Visser, J.D. et al.** A New Method for Measuring Angles After Total Hip Arthroplasty. *J. Bone Joint Surg. Br.,* 1981, vol. 63B, 556-559